# EUROPEAN PATENT APPLICATION

(11) **EP 3 181 608 A1**
(43) Date of publication of application: **21.06.2017**
(21) Application number: 16167206.8
(22) Date of filing: 27.04.2016
(51) Int. Cl.: C08G 59/42, C08G 59/68, C08G 63/19, C08G 63/50, C08J 5/24, B32B 15/092, H05K 1/03

(54) **AN EPOXY RESIN CURING AGENT AND PREPARATION METHODS AND USES THEREOF**

(30) Priority: 15.12.2015 CN 201510936667
(71) Applicant: Guangdong Guangshan New Materials Co., Ltd., Guangdong 523000 (CN)
(72) Inventor: PAN, Qingchong, 523000 Guangdong (CN)
(74) Representative: Brevalex

(57) **Abstract**

The present invention relates to an epoxy resin curing agent having a structure of Formula I and preparation methods and uses thereof. The present invention makes a resin composition formed by the epoxy resin curing agent have good low dielectric properties by using an epoxy resin curing agent having a specific structure, and the cured products of the epoxy resin composition have low dielectric constant and dielectric loss and good heat resistance and are low dielectric materials having great economic properties and being environmental friendly.

## Description

### Technical field

The present invention belongs to the technical field of low dielectric materials, especially relates to an epoxy resin curing agent and preparation methods and uses thereof.

### Background art

For the purpose of safety, electronic products represented by mobile phones, computers, video cameras and electronic game machines, household and office electrical products represented by air conditioners, refrigerators, television images, audio products etc., and various products used in other areas require low dielectric properties and heat resistance. In terms of electrical properties, the main factors needed to be considered also include the dielectric constant and dielectric loss of materials. Generally speaking, because the signal transmission speed of the substrate is inversely proportional to the square root of the dielectric constant of the substrate material, it is generally better that the dielectric constant of the substrate material is smaller; on the other hand, since the smaller dielectric loss represents the less loss of signal transmission, the transmission quality provided by the material with smaller dielectric loss is better.

Therefore, a problem urgently to be solved by suppliers of printed circuit board materials at the present stage is how to develop materials with low dielectric constant and low dielectric loss and apply them to the preparation of high frequency printed circuit boards.

### Contents of the invention

In view of this, the first purpose of the present invention is to provide an epoxy resin curing agent which has low dielectric properties, good heat resistance and mechanical properties and also has advantages of low cost.

In order to achieve the above purpose, the present invention utilizes the following technical solution:

An epoxy resin curing agent having a molecular structure as shown in Formula I:

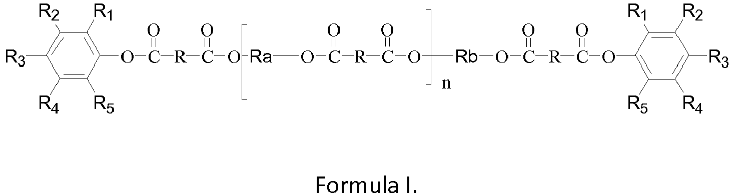

In Formula I, R is independently any organic group according with the chemical environment thereof; R₁, R₂, R₃, R₄ and R₅ are independently H or any organic group according with the chemical environment thereof; Rₐ, R_{b} are independently aromatic groups; n is an integer greater than or equal to zero. For example, n can be 0, 1, 2, 3, 4, 5, 6, 7, 8, 9, 10, etc., preferably 1-6.

R is any one of substituted or unsubstituted straight chain or branched alkyl, substituted or unsubstituted cycloalkyl, substituted or unsubstituted aryl, substituted or unsubstituted heteroaryl, substituted or unsubstituted alkylaryl, substituted or unsubstituted cycloalkylaryl, substituted or unsubstituted alkylheteroaryl or substituted or unsubstituted alkylheteroaryl;

Preferably, R₁, R₂, R₃, R₄ and R₅ are independently any one of H, substituted or unsubstituted straight chain or branched alkyl, substituted or unsubstituted cycloalkyl, substituted or unsubstituted aryl, substituted or unsubstituted heteroaryl, substituted or unsubstituted alkylaryl, substituted or unsubstituted cycloalkylaryl, substituted or unsubstituted alkylheteroaryl, substituted or unsubstituted alkoxy, substituted or unsubstituted cycloalkoxy, substituted or unsubstituted aryloxy, substituted or unsubstituted heteroaryloxy, substituted or unsubstituted arylalkoxy, substituted or unsubstituted alkylaryloxy, substituted or unsubstituted heteroarylalkoxy, substituted or unsubstituted alkylheteroaryloxy, substituted or unsubstituted carboxylate group, substituted or unsubstituted carbonate group, substituted or unsubstituted sulfonate group or substituted or unsubstituted phosphonate group, or a combination of at least two of them.

Preferably, Rₐ, R_{b} are independently any one of substituted or unsubstituted phenyl, substituted or unsubstituted biphenylyl, substituted or unsubstituted phenylalkyl, substituted or unsubstituted alkylphenyl, substituted or unsubstituted phenylalkylphenyl, substituted or unsubstituted pyridyl and substituted or unsubstituted furyl or substituted or unsubstituted morpholinyl.

Preferably, the epoxy resin curing agent has a molecular structure as shown in the following Formula II or III: 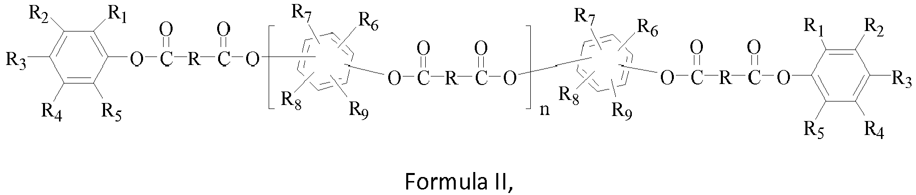 in Formula II, R is any organic group according with the chemical environment thereof; R₁, R₂, R₃, R₄, R₅, R₆, R₇, R₈ and R₉ are independently any organic group according with the chemical environment thereof; n is an integer greater than or equal to zero; For example, n can be 0, 1, 2, 3, 4, 5, 6, 7, 8, 9, 10, etc., preferably 1-6. 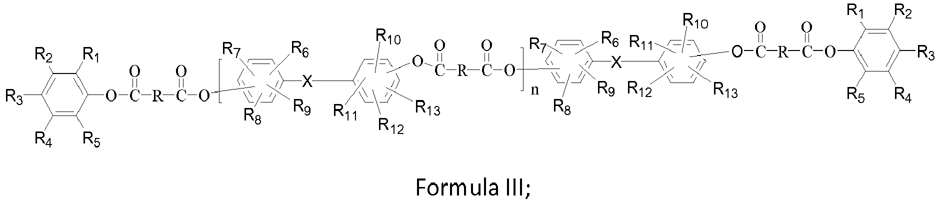 in Formula III, R is independently any organic group according with the chemical environment thereof; R₁, R₂, R₃, R₄, R₅, R₆, R₇, R₈, R₉, R₁₀, R₁₁, R₁₂ and R₁₃ are independently H or any organic group according with the chemical environment thereof; X is nothingness or any organic group according with the chemical environment thereof; n is an integer greater than or equal to zero; for example, n can be 0, 1, 2, 3, 4, 5, 6, 7, 8, 9, 10, etc., preferably 1-6.

In the present invention, said "according with the chemical environment thereof" means that the organic group can be linked to the adjacent atom to obtain a stable chemical bond.

In the present invention, in the fragment part of 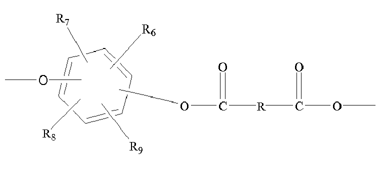 in Formula II, the two substituted positions in the benzene ring linked to oxygen can be para-position, ortho-position or meta-position, preferably meta-position.

Preferably, the epoxy resin curing agent has a molecular structure as shown in the following Formula IV or V: 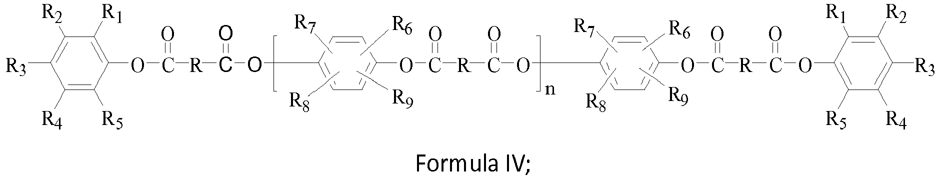 in Formula IV, R is any organic group according with the chemical environment thereof; R₁, R₂, R₃, R₄, R₅, R₆, R₇, R₈ and R₉ are independently H or any organic group according with the chemical environment thereof; n is an integer greater than or equal to zero; for example, n can be 0, 1, 2, 3, 4, 5, 6, 7, 8, 9, 10, etc., preferably 1-6. 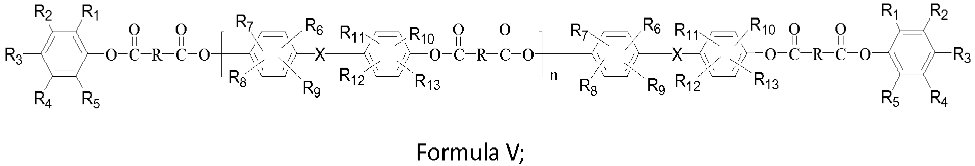 in Formula V, R is independently any organic group according with the chemical environment thereof; R₁, R₂, R₃, R₄, R₅, R₆, R₇, R₈, R₉, R₁₀, R₁₁, R₁₂ and R₁₃ are independently H or any organic group according with the chemical environment thereof; X is nothingness or any organic group according with the chemical environment thereof; n is an integer greater than or equal to zero; for example, n can be 0, 1, 2, 3, 4, 5, 6, 7, 8, 9, 10, etc., preferably 1-6.

Preferably, R is any one of substituted or unsubstituted straight chain or branched alkyl, substituted or unsubstituted cycloalkyl, substituted or unsubstituted aryl, substituted or unsubstituted heteroaryl, substituted or unsubstituted alkylaryl, substituted or unsubstituted cycloalkylaryl, substituted or unsubstituted alkylheteroaryl or substituted or unsubstituted alkylheteroaryl.

Specifically, R can be but not limited to -CH₂CH₂-, -CH₂CH₂CH₂-,

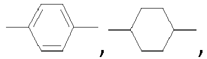

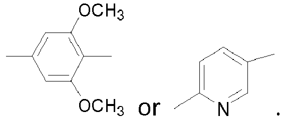

Preferably, R₁, R₂, R₃, R₄, R₅, R₆, R₇, R₈, R₉, R₁₀, R₁₁, R₁₂ and R₁₃ are independently any one of H, substituted or unsubstituted straight chain or branched alkyl, substituted or unsubstituted cycloalkyl, substituted or unsubstituted aryl, substituted or unsubstituted heteroaryl, substituted or unsubstituted alkylaryl, substituted or unsubstituted cycloalkylaryl, substituted or unsubstituted alkylheteroaryl, substituted or unsubstituted alkoxy, substituted or unsubstituted cycloalkoxy, substituted or unsubstituted aryloxy, substituted or unsubstituted heteroaryloxy, substituted or unsubstituted arylalkoxy, substituted or unsubstituted alkylaryloxy, substituted or unsubstituted heteroarylalkoxy, substituted or unsubstituted alkylheteroaryloxy, substituted or unsubstituted carboxylate group, substituted or unsubstituted carbonate group, substituted or unsubstituted sulfonate group or substituted or unsubstituted phosphonate group, or a combination of at least two of them.

In the present invention, the substituent is any organic group not containing halogen, such as alkyl, cycloalkyl, alkoxy, cycloalkoxy, aryl, heteroaryl, alkylaryl, heteroarylalkyl, carboxylate group, carbonate group, sulfonate group or phosphonate group and so on. Specifically, R₁, R₂, R₃, R₄, R₅, R₆, R₇, R₈, R₉, R₁₀, R₁₁, R₁₂ and R₁₃ are independently but not limited to any one of -CH₃, -CH₂CH₃, -OCH₃, -OCH₂CH₃, 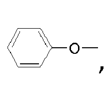 -SH, -SCH₃, -CONH₂, -COOH or -OOCCH₃, or a combination of at least two of them.

In the present invention, the substituted or unsubstituted straight chain or branched alkyl is preferably substituted or unsubstituted C1~C12 (for example C1, C2, C3, C4, C5, C6, C7, C8, C9, C10 or C11) straight chain or branched alkyl, further preferably C1-C8 straight chain or branched alkyl, and it is methyl when the carbon atom number is 1, and it is ethyl when the carbon atom number is 2.

The substituted or unsubstituted cycloalkyl is preferably substituted or unsubstituted C3-C12 (for example C4, C5, C6, C7, C8, C9, C10 or C11) cycloalkyl.

The substituted or unsubstituted alkoxy is preferably substituted or unsubstituted C1-C12 (for example C2, C3, C4, C5, C6, C7, C8, C9, C10 or C11) alkoxy.

The substituted or unsubstituted cycloalkoxy is preferably substituted or unsubstituted C3-C12 (for example C4, C5, C6, C7, C8, C9, C10 or C11) cycloalkoxy.

The substituted or unsubstituted aryl is preferably C7-C13 (for example C8, C9, C10, C11 or C12) alkylaryl; preferably is phenyl, naphthyl, 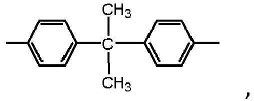 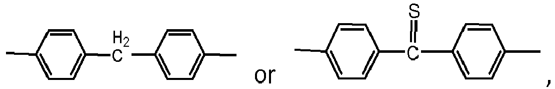 etc. Examples of phenyl include biphenylyl, terphenyl group, benzyl, phenethyl, or phenylpropyl, etc.

The substituted or unsubstituted heteroaryl is preferably C7-C13 (for example C8, C9, C10, C11or C12) heteroaralkyl, preferably is five or six membered heteroaryl, further preferably is substituted or unsubstituted furyl or pyridyl.

The substituted or unsubstituted alkylheteroaryl is preferably C7-C13 (for example C8, C9, C10, C11 or C12) alkylheteroaryl.

The substituted or unsubstituted aryloxy is preferably C7-C13 (for example C8, C9, C10, C11 or C12) aryloxy.

The substituted or unsubstituted heteroaryloxy is preferably C5-C13 (for example C6, C7, C8, C9, C10, C11 or C12) heteroaryloxy.

The substituted or unsubstituted arylalkoxy is preferably C7-C13 (for example C8, C9, C10, C11 or C12) arylalkoxy.

The substituted or unsubstituted alkylaryloxy is preferably C6-C13 (for example C7, C8, C9, C10, C11 or C12) alkylaryloxy.

The substituted or unsubstituted alkylheteroaryloxy is preferably C6-C13 (for example C7, C8, C9, C10, C11 or C12) alkylheteroaryloxy.

The term "substituted" used in the present invention means any one or more hydrogen atoms on a specified atom is/are substituted by a substituent selected from a specified group, with a condition that the specified atom does not exceed the normal valence thereof and the substituting result is forming a stable compound. When the substituent is an oxo group or a ketone group (i.e. =O), two hydrogen atoms on a specified atom are substituted. A ketone substituent does not exist on aromatic ring. "a stable compound" means a compound which can be separated robustly enough to an effective purity from the reaction mixture and be prepared into an effective compound.

Preferably, the epoxy resin curing agent of the present invention is one of compounds having the following structures or a combination of at least two of them: 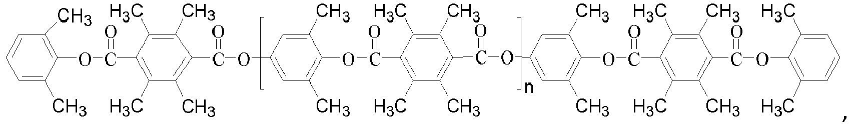 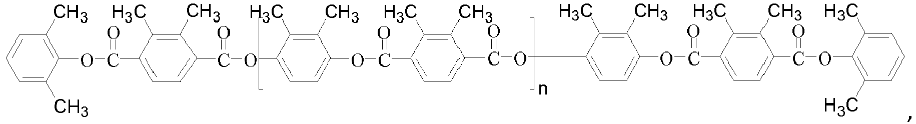 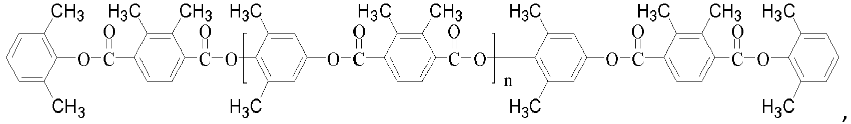 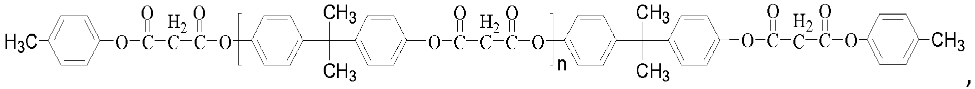 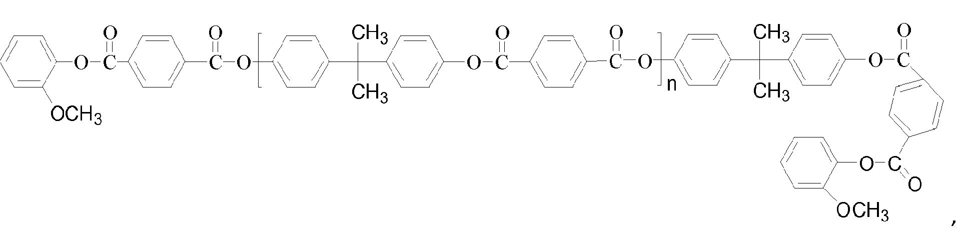 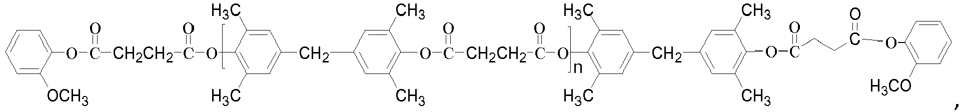 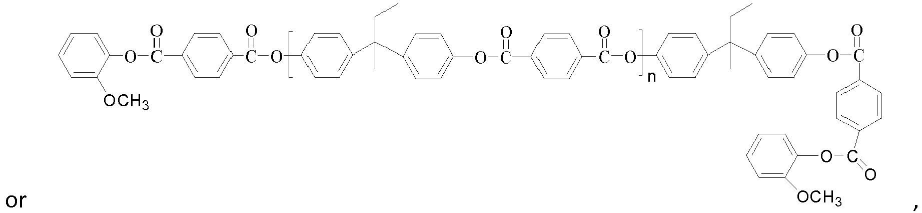 wherein n is an integer greater than or equal to zero; for example n can be 0, 1, 2, 3, 4, 5, 6, 7, 8, 9, 10, etc., preferably 1-6.

In another aspect, the invention provides a method for preparing the epoxy resin curing agent, and the epoxy resin curing agent prepared by the method has low dielectric properties, good heat resistance and mechanical properties.

A method for preparing the epoxy resin curing agent, which reacts phenolic compounds and poly(acyl chloride) compounds or polybasic acid compounds to obtain the epoxy resin curing agent.

In the present invention, poly(acyl chloride) compounds are binary acyl chloride compounds or poly(acyl chloride) compounds having more than two acyl chloride groups. The polybasic acid compounds are binary or polybasic acid compounds. For example, typically, in the present invention, a corresponding ester is obtained by conducting an esterification by reacting a phenolic compound and a binary acid compound using concentrated sulfuric acid or solid acid as a catalyst. A corresponding ester can also be obtained by reacting a phenolic compound and acyl chloride with removing hydrogen chloride.

The preparation method of the ester type epoxy resin curing agent provided by the present invention is a known method for preparing esters, and there is no special provision to this in the present invention. For example, the novel ester type epoxy resin compound of the present invention can be obtained by the following steps: in molten or dissolved binary acid or polybasic acid compounds, using common dehydration agent such as sulfuric acid, phosphoric acid, solid acid compounds and others as a catalyst, or using binary acyl chloride or poly(acyl chloride) compounds to replace binary acid or polybasic acid compounds, using common catalysts for removing hydrogen chloride, stirring the mixture while adding phenolic compounds at a certain temperature, and removing solvents, by-products or other impurities by physical methods after the dehydration polymerization is completed.

Said phenolic compounds means phenolic compounds corresponding to Rₐ or R_{b} group in Formula I, for example the phenolic compounds having Rₐ or R_{b}, can be dihydroxybenzene, which can serve as hydroquinone, catechol and resorcinol.

Said binary acid refers to dicarboxylic acid having R group in Formula I, that is HOOC-R-COOH Said phenolic compound is condensed with dicarboxylic acid to obtained an ester, and thus obtain the epoxy resin curing agent as show in Formula I. Said reaction and the needed reaction conditions are known by those skilled in the art.

In another aspect, the present invention provides an epoxy resin composition having low dielectric properties, good heat resistance and good mechanical properties.

The epoxy resin composition comprises an epoxy resin and the epoxy resin curing agent described above.

Preferably, the epoxy resin is any one of liquid bisphenol A epoxy resin, liquid bisphenol F epoxy resin, solid bisphenol A epoxy resin, solid bisphenol F epoxy resin, bisphenol S epoxy resin, cyclopentadiene epoxy resin or biphenyl epoxy resin, or a combination of at least two of them.

Preferably, the epoxy resin composition also comprises a curing accelerator. Preferably, the curing accelerator is any one of imidazole curing accelerators, organophosphine curing accelerators, tertiary amine curing accelerators, quaternary ammonium salts, DBU and derivatives thereof, pyridines and derivatives thereof, or a mixture of at least two of them.

Preferably, the epoxy resin composition also comprises an inorganic filler.

Preferably, the inorganic filler is any one selected from the group consisting of aluminum hydroxide, boehmite, silica, talcum powder, mica, barium sulfate, lithopone, calcium carbonate, wollastonite, kaolin, brucite, diatomite, bentonite or pumice powder, or a mixture of at least two of them.

Preferably, the epoxy resin composition also comprises a flame retardant which is an organic flame retardant and/or an inorganic flame retardant known in the art.

The above epoxy resin curing agent can be manufactured into a molding compound and a prepreg according to the actual needs.

A molding compound comprising the epoxy resin composition of the present invention.

A prepreg prepared by impregnating a substrate with the above epoxy resin composition or coating the above epoxy resin composition onto a substrate.

The substrate can be a glass fiber substrate, a polyester substrate, a polyimide substrate, a ceramic substrate or a carbon fiber substrate, etc.

Here, the specific process conditions of impregnation or coating are not particularly limited. The "prepreg" is "bonding sheet" well-known by those skilled in the art.

A composite metal laminate comprising more than one sheet of the prepregs described above and prepared by coating metal layer on the surface of the prepregs, overlapping and pressing successively.

Here, the material of the surface-coated metal layer is aluminium, copper, iron and alloys of any combination thereof.

Specific examples of the composite metal laminate are CEM-1 copper clad laminate, CEM-3 copper clad laminate, FR-4 copper clad laminate, FR-5 copper clad laminate, CEM-1 aluminum clad laminate, CEM-3 aluminum clad laminate, FR-4 aluminum clad laminate or FR-5 aluminum clad laminate.

A wiring board prepared by processing wires on the surface of the composite metal laminate as described above.

The raw materials of the epoxy resin composition form a coating having good low dielectric properties on the composite metal laminate by curing, and this can improve the wide use of the wiring board in industries of machine, equipment, instrument, meter, etc. which need wiring board, for example electronic industry, electrical and electrical appliance industry, transportation industry, aerospace industry, toy industry, etc.

The above term "xxxyl or group " refers to the remaining parts of the molecular structure of xxx compounds after one or more hydrogen atoms or other atoms or atomic groups are removed.

The present invention utilizes an epoxy resin curing agent having a specific structure and make a resin composition formed by the epoxy resin curing agent have good low dielectric properties, and the cured products thereof have good heat resistance, mechanical properties and low dielectric constant and dielectric loss, which is also a low dielectric material having great economic properties and being environmental friendly. A copper clad laminate prepared by using the resin composition has a dielectric constant (1GHz) of 3.25-3.32, a dielectric loss (1GHz) of 0.005-0.006, a Tg which can be up to more than 158°C, a T-peeling strength which can be up to more than 1.97 kg/mm², an interlaminar peeling strength which can be up to more than 1.62kg/mm² and a saturated water absorption which can be less than 0.34%.

### Embodiments

The technical solution of the present invention is further described by the following examples.

In the following examples, all the raw materials can be obtained through commercial purchase.

### Example 1

The epoxy resin curing agent of the present example has the following structure:

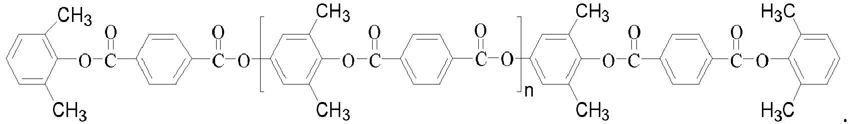

The preparation method thereof is:
1 mol of 2,6-dimethylhydroquinone was added into a 1000 mL glass reactor equipped with a stirrer using dichloromethane as solvent; the mixture was stirred and dissolved, and then 3 mL of 98% concentrated sulfuric acid was added as a catalyst; then a solution of terephthalic acid (1.1 mol) in dichloromethane was slowly dripped; the mixture was stirred at room temperature for 10 hours after the drip was completed; then 1.1 mol of o-dimethylphenol was added and the mixture was continued to stir for 8 hours; the by-products in the system were removed by physical methods and the solvent were removed by evaporation, and then 100 g of objective product with an ester equivalent of 100 g/eq was obtained; this objective product was named as epoxy resin curing agent A.

The obtained epoxy resin curing agent A was characterized by nuclear magnetic resonance hydrogen spectrum, and the results are as follows:
¹H NMR (CDCl₃, 500 MHz): δ ppm 6.92-6.96 (m, hydrogen on benzene ring in 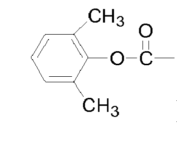 ), 6.81-6.83 (m, hydrogen on benzene ring in 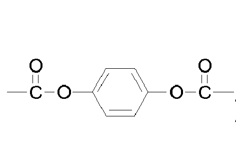
), 2.32 (s, CH₃).

Characteristic peak positions in infrared spectroscopy: ester carbonyl, 1730-1740 cm-¹; C-O-C in ester group, 1200 cm-¹; ortho-position substituted benzene ring, 750 cm-¹; para-position substituted benzene ring, 860-790 cm-¹; absorption peak of methyl, 2995cm⁻¹.

An epoxy resin composition is prepared by using the epoxy resin curing agent and other components, and a copper clad laminate is prepared by using the epoxy resin composition, and the steps thereof are as follows:
136 g of the above epoxy resin curing agent and 0.1 g of curing accelerator DBU were added into 100 g of bisphenol-A epoxy resin with an epoxide equivalent of 187 g/eq and 100 g of o-cresol novolac epoxy resin with an epoxide equivalent of 120 g/eq. A copper clad laminate meeting the national, UL and other standards is prepared by using the epoxy resin composition according to generally used copper clad laminate production process. The copper clad laminate is named as copper clad laminate a and the properties thereof are measured and the results are shown in table 1.

### Example 2

The epoxy resin curing agent of the present example has the following structure:

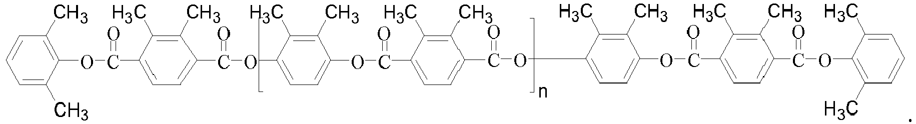

The preparation method thereof is:
1 mol of 2,6-dimethylhydroquinone was added into a 1000 mL glass reactor equipped with a stirrer; the mixture was stirred and dissolved, and then 3 mL of 98% concentrated sulfuric acid was added as a catalyst; then 1.1 mol of 2,3-dimethylterephthalic acid was slowly dripped; the mixture was stirred at room temperature for 15 hours after the drip was completed; then 1.2 mol of o-dimethylphenol was added and the mixture was continued to stir for 6 hours; the by-products in the system were removed by physical methods and the solvent were removed by evaporation, and then 180 g of objective product with an ester equivalent of 180 g/eq was obtained; this objective product was named as epoxy resin curing agent B.

The obtained epoxy resin curing agent B was characterized by nuclear magnetic resonance hydrogen spectrum, and the results are as follows:
¹H NMR (CDCl₃, 500 MHz): δ ppm 7.7-7.9 (m, hydrogen on benzene ring in 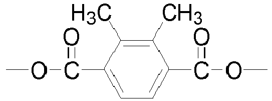 ), 6.91-6.94 (m, hydrogen on benzene ring in 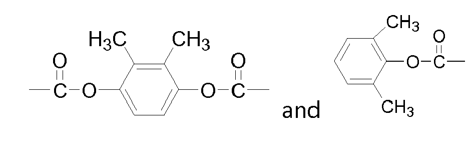 ), 2.32 (s, CH₃).
Characteristic peak positions in infrared spectroscopy: ester carbonyl, 1730-1740 cm-¹; C-O-C in ester group, 1200 cm-¹; ortho-position substituted benzene ring, 750 cm-¹; para-position substituted benzene ring, 860-790 cm-¹; absorption peak of methyl, 2995cm⁻¹.

An epoxy resin composition is prepared by using the epoxy resin curing agent and other components, and a copper clad laminate is prepared by using the epoxy resin composition, and the steps thereof are as follows:
245 g of the above epoxy resin curing agent and 0.1 g of curing accelerator DBU were added into 100 g of bisphenol-A epoxy resin with an epoxide equivalent of 187 g/eq and 100 g of o-cresol novolac epoxy resin with an epoxide equivalent of 120 g/eq. A copper clad laminate meeting the national, UL and other standards is prepared by using the epoxy resin composition according to generally used copper clad laminate production process. The copper clad laminate is named as copper clad laminate b and the properties thereof are measured and the results are shown in table 1.

### Example 3

The epoxy resin curing agent of the present example has the following structure:

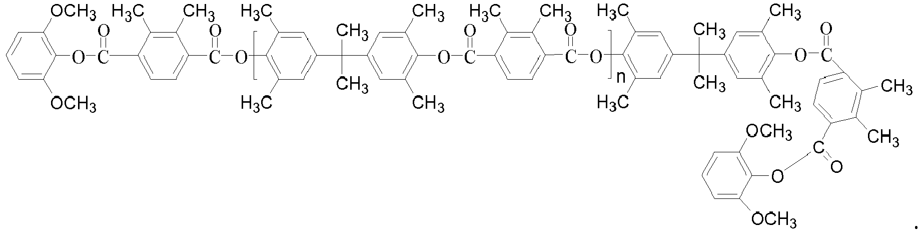

The preparation method thereof is:
1 mol of 2,6,2',6'-tetramethyl bisphenol-A was added into a 1000 mL glass reactor equipped with a stirrer using dichloromethane as solvent; the mixture was stirred and dissolved, and then 3 mL of 98% concentrated sulfuric acid was added as a catalyst; then a solution of 2,3-dimethylterephthalic acid (1.1 mol) in dichloromethane was slowly dripped; the mixture was stirred at room temperature for 10 hours after the drip was completed; then 1.2 mol of o-dimethylphenol was added and the mixture was continued to stir for 8 hours; the by-products in the system were removed by physical methods and the solvent were removed by evaporation, and then 220 g of objective product with an ester equivalent of 220 g/eq was obtained; this objective product was named as epoxy resin curing agent C.

The obtained epoxy resin curing agent C was characterized by nuclear magnetic resonance hydrogen spectrum, and the results are as follows:
¹H NMR (CDCl₃, 500 MHz): δ ppm 7.7-7.9 (m, hydrogen on benzene ring in 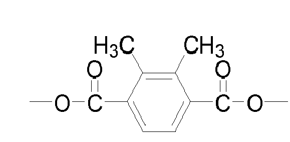 ), 6.4-6.9 (m, hydrogen on benzene ring in 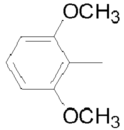 and 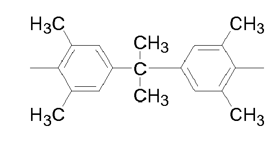 ), 3.7 (m, hydrogen on methyl in 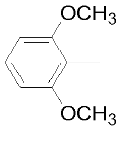 ), 2.35 (s, hydrogen on methyl connected to benzene ring), 1.65 (m, hydrogen on methyl which is not connected to benzene ring in 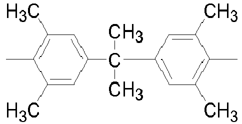 group).

Characteristic peak positions in infrared spectroscopy: ester carbonyl, 1730-1740 cm-¹; C-O-C in ester group, 1200 cm-¹; ortho-position substituted benzene ring, 750 cm-¹; skeleton vibration absorption peak of benzene ring in 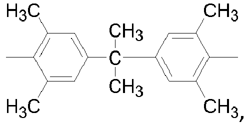 1611 cm⁻¹, 1509 cm-¹ , 1446 cm-¹; bending vibration absorption peak of -CH₃ in 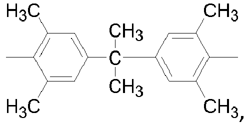 1435 cm-¹, 1382 cm⁻¹.

An epoxy resin composition is prepared by using the epoxy resin curing agent and other components, and a copper clad laminate is prepared by using the epoxy resin composition, and the steps thereof are as follows:
299 g of the above epoxy resin curing agent and 0.1 g of curing accelerator DBU were added into 100 g of bisphenol-A epoxy resin with an epoxide equivalent of 187 g/eq and 100 g of o-cresol novolac epoxy resin with an epoxide equivalent of 120 g/eq. A copper clad laminate meeting the national, UL and other standards is prepared by using the epoxy resin composition according to generally used copper clad laminate production process. The copper clad laminate is named as copper clad laminate c and the properties thereof are measured and the results are shown in table 1.

### Example 4

The epoxy resin curing agent of the present example has the following structure:

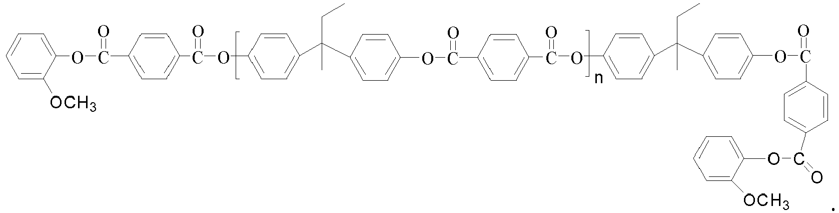

The preparation method thereof is:
1 mol of bisphenol-B was added into a 1000 mL glass reactor equipped with a stirrer using dichloromethane as solvent; the mixture was stirred and dissolved, and then 3 mL of 98% concentrated sulfuric acid was added as a catalyst; then a solution of terephthaloyl chloride (1.1 mol) in dichloromethane was slowly dripped; the mixture was stirred at room temperature for 10 hours after the drip was completed; then 1.2 mol of o-dimethylphenol was added and the mixture was continued to stir for 8 hours; the by-products in the system were removed by physical methods and the solvent were removed by evaporation, and then 190 g of objective product with an ester equivalent of 190 g/eq was obtained; this objective product was named as epoxy resin curing agent D.

The obtained epoxy resin curing agent D was characterized by nuclear magnetic resonance hydrogen spectrum, and the results are as follows:
¹H NMR (CDCl₃, 500 MHz): δ ppm 8.29 (s, hydrogen on benzene ring in 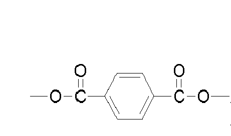 ), 7.1-7.2 (m, hydrogen on benzene ring in 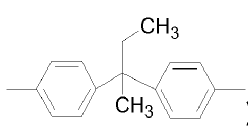 ), 6.8-6.9 (m, 8H, hydrogen on benzene ring in 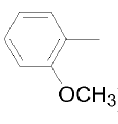 ), 3.7 (m, hydrogen on methyl in 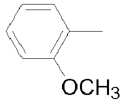 group), 1.91 (m, hydrogen on methylene in 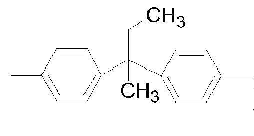 ), 1.72 (m, hydrogen on methyl connected to quaternary carbon atom in 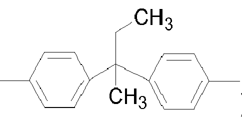 ), 1.0 (m, hydrogen on methyl connected to methylene in 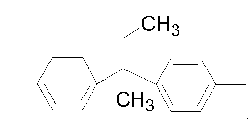 ).

Characteristic peak positions in infrared spectroscopy: ester carbonyl, 1730-1740 cm-¹; C-O-C in ester group, 1200 cm-¹; ortho-position substituted benzene ring, 750 cm-¹; skeleton vibration absorption peak of benzene ring in 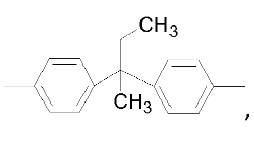 1641 cm-¹, 1500 cm-¹, 1446 cm-¹; bending vibration absorption peak of -CH₃, 1435 cm-¹, 1382 cm-¹; absorption peak of methyl ether, 2995cm⁻¹.

An epoxy resin composition is prepared by using the epoxy resin curing agent and other components, and a copper clad laminate is prepared by using the epoxy resin composition, and the steps thereof are as follows:
258 g of the above epoxy resin curing agent and 0.1 g of curing accelerator DBU were added into 100 g of bisphenol-A epoxy resin with an epoxide equivalent of 187 g/eq and 100 g of o-cresol novolac epoxy resin with an epoxide equivalent of 120 g/eq. A copper clad laminate meeting the national, UL and other standards is prepared by using the epoxy resin composition according to generally used copper clad laminate production process. The copper clad laminate is named as copper clad laminate d and the properties thereof are measured and the results are shown in table 1.

### Comparative Example 1

In the present comparative example, a commonly used linear phenolic resin with a phenolic hydroxyl equivalent of 105g/eq was used to replace the epoxy resin curing agent of the present application as a curing agent, and a method same as that in example 1 was used to prepare a copper clad laminate e. The properties of the copper clad laminate e are measured and the results are shown in table 1.

### Comparative Example 2

100 g of commercially available MDI-modified epoxy resin with an epoxide equivalent of 380.0 g/eq, 57.9 g of resin compound having a structure of Formula (I) with a ester equivalent of 220 and 0.2 g of pyridine were dissolved in appropriate acetone to form a solution. A copper clad laminate was prepared by using standard glass fiber cloth according to a well-known method. The copper clad laminate is named as copper clad laminate f. The measured properties of the copper clad laminate f are shown in Table 1.

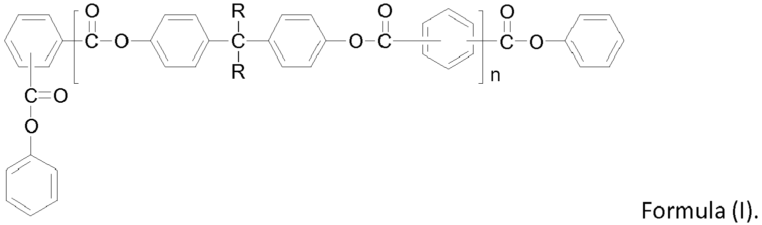

The test results of products of examples and comparative examples are shown in table 1 (the specific test methods are not described considering that they are well-known by those skilled in the art).

**Table 1**

| Test Item | Copper clad laminate a | Copper clad laminate b | Copper clad laminate c | Copper clad laminate d | Copper clad laminate e | Copper clad laminate f |
|---|---|---|---|---|---|---|
| Tg (DSC) (°C) | 159 | 165 | 158 | 160 | 143 | 146 |
| T-peeling strength (kg/mm² ) | 1,97 | 2,03 | 2,10 | 2,0 | 1,45 | 1,53 |
| Interlaminar peeling strength (kg/mm² ) | 1,65 | 1,63 | 1,75 | 1,62 | 1,1 | 0,80 |
| Saturated water absorption (%) | 0,32 | 0,31 | 0,34 | 0,32 | 0,35 | 0,58 |
| Dielectric constant (1GHz) | 3,32 | 3,25 | 3,28 | 3,25 | 4,68 | 4,52 |
| Dielectric loss (1GHz) | 0,005 | 0,006 | 0,0057 | 0,0059 | 0,11 | 0,12 |

As can be seen from table 1, the copper clad laminates of the present invention prepared by using an resin composition prepared by using a compound having a structure of Formula I as a curing agent have a dielectric constant (1GHz) of 3.25-3.32, a dielectric loss (1GHz) of 0.005-0.006, a Tg which can be up to more than 158°C, a T-peeling strength which can be up to more than 1.97 kg/mm², an interlaminar peeling strength which can be up to more than 1.62 kg/mm² and a saturated water absorption which can be less than 0.34%, which are much better than the properties of the copper clad laminates of comparative examples.

The applicant states that: the present invention illustrates the epoxy resin curing agent of the present invention and the preparation methods and uses thereof by the above examples, but the present invention is not limited to the above examples, that is, it does not mean that the present invention must be conducted relying on the above examples. Those skilled in the art should understand that any modification to the present invention, any equivalent replacement of each raw material of the product of the present invention and the addition of auxiliary ingredient, the selection of specific embodiment and the like all fall into the protection scope and the disclosure scope of the present invention.

## Claims

1. An epoxy resin curing agent, **characterized in that**, it has a molecular structure as shown in Formula I: 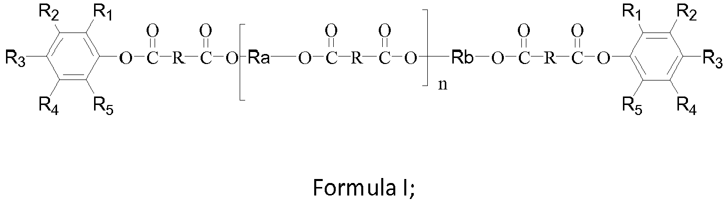 wherein, R is independently any organic group according with the chemical environment thereof; R₁, R₂, R₃, R₄ and R₅ are independently H or any organic group according with the chemical environment thereof; Rₐ, R_{b} are independently aromatic groups; n is an integer greater than or equal to zero.

2. The epoxy resin curing agent of claim 1, **characterized in that**, R is any one of substituted or unsubstituted straight chain or branched alkyl, substituted or unsubstituted cycloalkyl, substituted or unsubstituted aryl, substituted or unsubstituted heteroaryl, substituted or unsubstituted alkylaryl, substituted or unsubstituted cycloalkylaryl, substituted or unsubstituted alkylheteroaryl or substituted or unsubstituted alkylheteroaryl;
Preferably, R₁, R₂, R₃, R₄ and R₅ are independently any one of H, substituted or unsubstituted straight chain or branched alkyl, substituted or unsubstituted cycloalkyl, substituted or unsubstituted aryl, substituted or unsubstituted heteroaryl, substituted or unsubstituted alkylaryl, substituted or unsubstituted cycloalkylaryl, substituted or unsubstituted alkylheteroaryl, substituted or unsubstituted alkoxy, substituted or unsubstituted cycloalkoxy, substituted or unsubstituted aryloxy, substituted or unsubstituted heteroaryloxy, substituted or unsubstituted arylalkoxy, substituted or unsubstituted alkylaryloxy, substituted or unsubstituted heteroarylalkoxy, substituted or unsubstituted alkylheteroaryloxy, substituted or unsubstituted carboxylate group, substituted or unsubstituted carbonate group, substituted or unsubstituted sulfonate group or substituted or unsubstituted phosphonate group; Preferably, Rₐ, R_{b} are independently any one of substituted or unsubstituted phenyl, substituted or unsubstituted biphenylyl, substituted or unsubstituted phenylalkyl, substituted or unsubstituted alkylphenyl, or substituted or unsubstituted phenylalkylphenyl.

3. The epoxy resin curing agent of claim 1 or 2, **characterized in that**, the epoxy resin curing agent has a molecular structure as shown in the following Formula II or III: 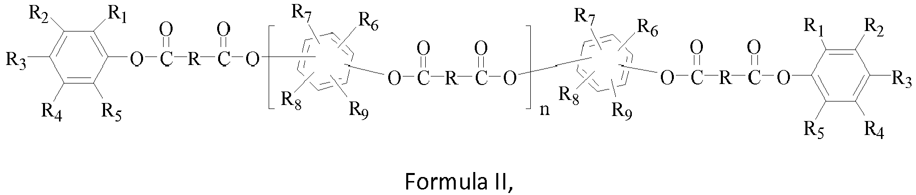 in Formula II, R is any organic group according with the chemical environment thereof; R₁, R₂, R₃, R₄, R₅, R₆, R₇, R₈ and R₉ are independently any organic group according with the chemical environment thereof; n is an integer greater than or equal to zero; 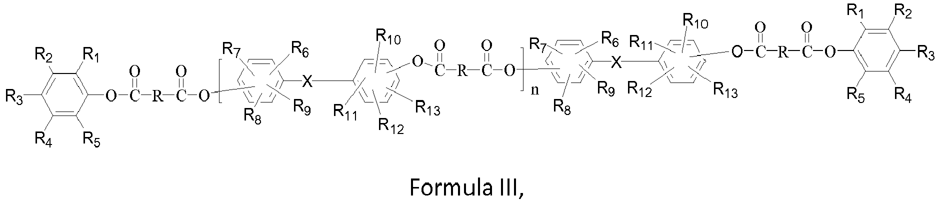 in Formula III, R is any organic group according with the chemical environment thereof; R₁, R₂, R₃, R₄, R₅, R₆, R₇, R₈, R₉, R₁₀, R₁₁, R₁₂ and R₁₃ are independently H or any organic group according with the chemical environment thereof; X is nothingness or any organic group according with the chemical environment thereof; n is an integer greater than or equal to zero;
Preferably, the epoxy resin curing agent has a molecular structure as shown in the following Formula IV or V: 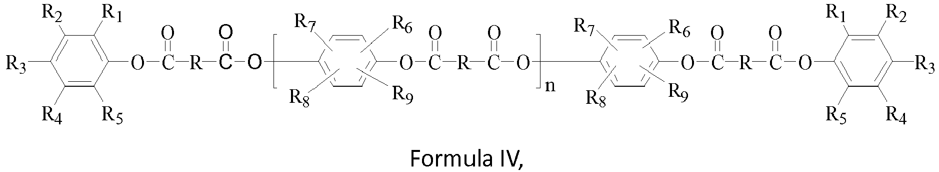 in Formula IV, R is any organic group according with the chemical environment thereof; R₁, R₂, R₃, R₄, R₅, R₆, R₇, R₈ and R₉ are independently H or any organic group according with the chemical environment thereof; n is an integer greater than or equal to zero; 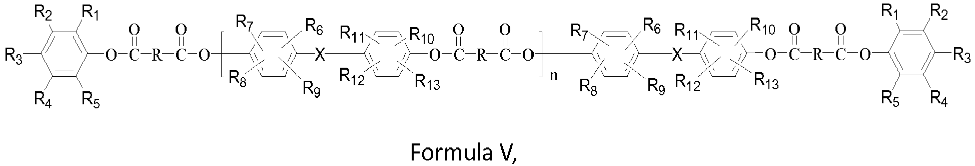 in Formula V, R is independently any organic group according with the chemical environment thereof; R₁, R₂, R₃, R₄, R₅, R₆, R₇, R₈, R₉, R₁₀, R₁₁, R₁₂ and R₁₃ are independently H or any organic group according with the chemical environment thereof; X is nothingness or any organic group according with the chemical environment thereof; n is an integer greater than or equal to zero;
Preferably, R is any one of substituted or unsubstituted straight chain or branched alkyl, substituted or unsubstituted cycloalkyl, substituted or unsubstituted aryl, substituted or unsubstituted heteroaryl, substituted or unsubstituted alkylaryl, substituted or unsubstituted cycloalkylaryl, substituted or unsubstituted alkylheteroaryl or substituted or unsubstituted alkylheteroaryl;
Preferably, R₁, R₂, R₃, R₄, R₅, R₆, R₇, R₈, R₉, R₁₀, R₁₁, R₁₂ and R₁₃ are independently any one of H, substituted or unsubstituted straight chain or branched alkyl, substituted or unsubstituted cycloalkyl, substituted or unsubstituted aryl, substituted or unsubstituted heteroaryl, substituted or unsubstituted alkylaryl, substituted or unsubstituted cycloalkylaryl, substituted or unsubstituted alkylheteroaryl, substituted or unsubstituted alkoxy, substituted or unsubstituted cycloalkoxy, substituted or unsubstituted aryloxy, substituted or unsubstituted heteroaryloxy, substituted or unsubstituted arylalkoxy, substituted or unsubstituted alkylaryloxy, substituted or unsubstituted heteroarylalkoxy, substituted or unsubstituted alkylheteroaryloxy, substituted or unsubstituted carboxylate group, substituted or unsubstituted carbonate group, substituted or unsubstituted sulfonate group or substituted or unsubstituted phosphonate group, or a combination of at least two of them.

4. The epoxy resin curing agent of any one of claims 1-3, **characterized in that**, the epoxy resin curing agent is one of compounds having the following structures or a combination of at least two of them:

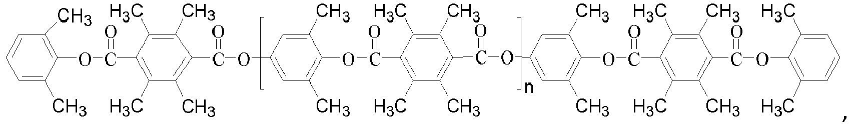

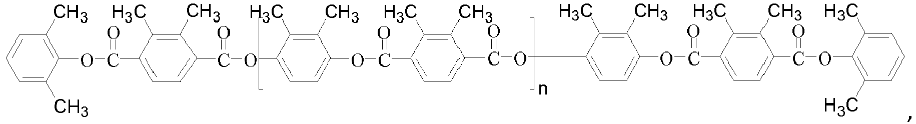

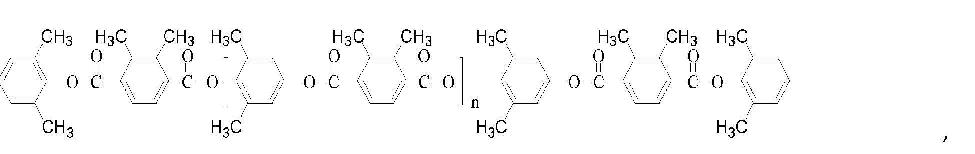

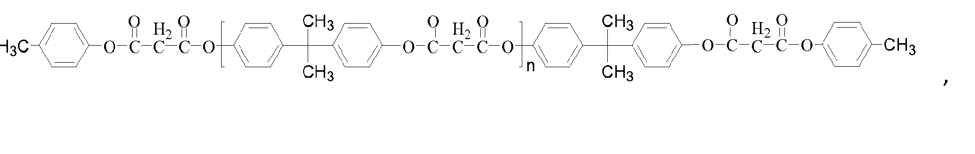

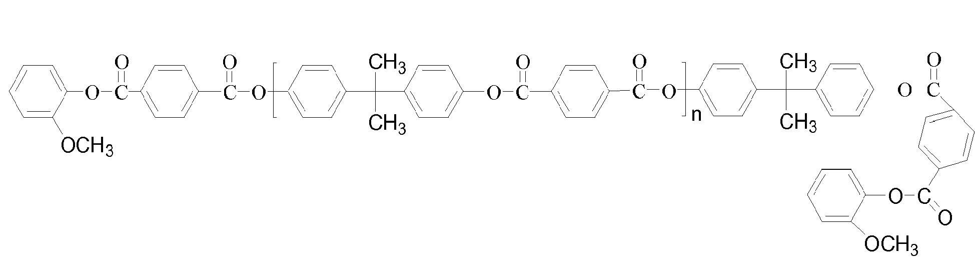

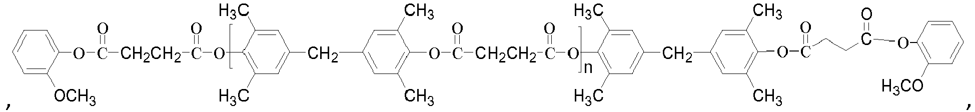

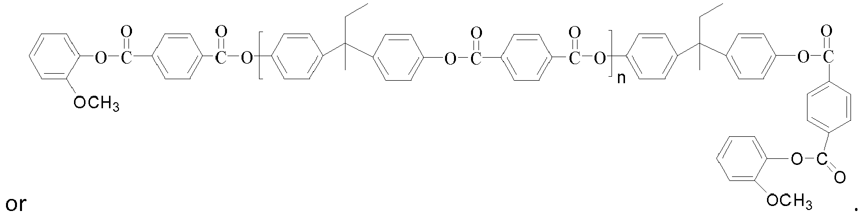

5. An epoxy resin composition, **characterized in that**, the epoxy resin composition comprises an epoxy resin and the epoxy resin curing agent of any one of claims 1-4; preferably, the epoxy resin is any one of liquid bisphenol A epoxy resin, liquid bisphenol F epoxy resin, solid bisphenol A epoxy resin, solid bisphenol F epoxy resin, bisphenol S epoxy resin, cyclopentadiene epoxy resin or biphenyl epoxy resin, or a combination of at least two of them;
preferably, the epoxy resin composition also comprises a curing accelerator;
preferably, the curing accelerator is any one of imidazole curing accelerators, organophosphine curing accelerators, tertiary amine curing accelerators, quaternary ammonium salts, DBU and derivatives thereof, pyridines and derivatives thereof, or a mixture of at least two of them;
preferably, the epoxy resin composition also comprises an inorganic filler;
preferably, the epoxy resin composition also comprises a flame retardant.

6. A prepreg, **characterized in that**, it is prepared by impregnating a substrate with the epoxy resin composition of claim 5 or coating the epoxy resin composition of claim 5 onto a substrate.

7. The prepreg of claim 6, **characterized in that**, the substrate is a glass fiber substrate, a polyester substrate, a polyimide substrate, a ceramic substrate or a carbon fiber substrate.

8. A composite metal laminate, **characterized in that**, it comprises more than one sheet of the prepregs of claim 6 or 7 and is prepared by coating metal layer on the surface of the prepregs, overlapping and pressing successively.

9. The composite metal laminate of claim 8, **characterized in that**, the material of the surface-coated metal layer is aluminium, copper, iron and alloys of any combination thereof;
preferably, the composite metal laminate are CEM-1 copper clad laminate, CEM-3 copper clad laminate, FR-4 copper clad laminate, FR-5 copper clad laminate, CEM-1 aluminum clad laminate, CEM-3 aluminum clad laminate, FR-4 aluminum clad laminate or FR-5 aluminum clad laminate.

10. A wiring board, **characterized in that**, it is prepared by processing wires on the surface of the composite metal laminate of claim 8 or 9.
